# EUROPEAN PATENT SPECIFICATION

(11) **EP 4 031 105 B1**
(45) Date of publication and mention of the grant of the patent: **08.11.2023**
(21) Application number: 20781304.9
(22) Date of filing: 10.09.2020
(51) Int. Cl.: A61K 8/60, A61K 8/85, A61K 8/92, A61K 8/86, A61Q 19/00, A61K 8/39, A61Q 19/10

(54) **STABILIZER CONCENTRATES FOR WAX DISPERSIONS**
STABILISATORKONZENTRATE FÜR WACHSDISPERSIONEN
CONCENTRÉS DE STABILISATEUR DE DISPERSIONS DE CIRE

(30) Priority: 18.09.2019 EP 19198065
(43) Date of publication of application: 27.07.2022
(73) Proprietor: BASF SE, 67056 Ludwigshafen am Rhein (DE)
(72) Inventor: NIEENDICK, Claus, 40589 Düsseldorf (DE); MAUER, Werner, 40589 Düsseldorf (DE); BECKER, Anke, 40589 Düsseldorf (DE); BOYXEN, Norbert, 40789 Monheim (DE)
(74) Representative: BASF IP Association
(86) International application number: PCT/EP2020/075321
(87) International publication number: WO 2021/052856

(56) References cited:
- DE-A1-102015 213 478
- US-A- 5 474 776
- US-A1- 2015 297 485
- DATABASE GNPD [Online] MINTEL; 19 March 2015 (2015-03-19), anonymous: "Elemental Facial Barrier Cream", XP055669714, retrieved from www.gnpd.com Database accession no. 3070879
- MATSUDA ET AL: "Cosmetic pencils containing wax and fatty acid sugar esters", CAPLUS, 17 December 1985 (1985-12-17), XP002173380,

## Description

### Field of the invention

This invention relates to stabilizer concentrates which are able to improve the stability of mono- and/or diesters of mono- and/or diethylene glycol waxes, which are used in form of a wax dispersion in personal care dispersions, wherein the stabilizer concentrates comprising hydrogenated castor oil and nonionic emulsifiers and are free of anionic surfactants; additionally, the invention relates to the use of such concentrates and a process for their production and personal care compositions containing them and a process for the production of the personal care compositions.

### Prior Art

The use of wax dispersions based on alkylene glycol fatty acid esters is a long-established and known technique for providing personal care products with an attractive, rich and interesting appearance. Depending on the desired appearance properties wax dispersions can be modified to cause pearlizing (pearlescent) properties with high brilliance and/or an opacifier properties with a high degree of whiteness. For their intended use in personal care compositions the wax dispersions need to have a high compatibility with other ingredients, a good physical stability, especially storage stability and a high performance in the appearance properties. Additionally, the producers of personal care compositions want to have wax dispersions, which are easily to add within the process, without a time and energy consuming production step like melting.

A favourite option is to use of the wax in form of a premixed wax dispersion. European Patents EP 0581193 B1 and EP 0568848 B1 disclose flowable concentrated pearlizing formulations containing large quantities of fatty acid glycol esters, betaines as zwitterionic surfactants and fatty alcohol alkoxylates as nonionic surfactants. The International patent application WO 96/21711 also describes pearlizing concentrates containing nonionic, zwitterionic and anionic surfactants.

WO 2003/033634 A1 discloses opacifier preparations based on fatty acid glycol esters, amphoteric surfactants and fatty acid partial glycerides, which are flowable and high concentrated.

Unfortunately, the use of wax dispersions in personal care products sometimes leads to separation of the product, which is at least quite aesthetically unattractive to customers. In order to improve the stability of personal care compositions containing wax dispersions EP 2037877 B1 discloses to use hydrogenated castor oil. However, the hydrogenated castor oil needs to be warmed up in an additional manufacturing step, which is slow and costly.

EP 3324927 describes an aqueous dispersion of hydrogenated castor oil, which was used together with a surfactant mixture consisting of anionic, amphoteric and nonionic surfactants to stabilize cosmetic compositions like shampoos or shower gels. The dispersion can be added at room temperature, i.e. no additional manufacturing step is necessary. Nevertheless, these dispersions of hydrogenated castor oil have high viscosities and are not flowable, therefore a long time of stirring and high steering rates are necessary for homogenization.

WO 2016/064847 describes personal care compositions comprising a premix composition with crystals from hydrogenated castor oil. The premix is formed by combining hydrogenated castor oil with surfactants and water and heating all up to 65 to 84°C under high shear. However, according to all examples the premix compositions need the presence of anionic surfactants and show high viscosities. Additionally, the production of such dispersions seems to be extremely temperature sensitive as temperatures above 84°C do not lead to the desired result.

Accordingly, there is a need for further stabilizing agents for waxes used in form of a wax dispersion in the personal care composition with a high active content (concentrates) and very low viscosities. Additionally, the stabilizing agents should improve the stability of the wax in the personal care composition, especially in personal care compositions containing deposition agents, e.g. in conditioners containing cationic polymers Furthermore, there is a need for stabilizing agents which can be blended in personal care compositions in an easy and cost-efficient manner, especially at room temperature ("via cold processing").

### Description of the invention

The invention relates to stabilizer concentrates for waxes selected from the group consisting of mono- and/or diesters of mono- and/or diethylene glycol esters, whereby the waxes are in form of a dispersion comprised in personal care compositions, and wherein the concentrates containing
(a) hydrogenated castor oil,
(b) nonionic emulsifiers selected from the group consisting of fatty acid polyglycol ester; fatty acid polyglycol ether, ethoxylated fatty acid glycerol ester; mixed ethers or mixed formals; polysorbates and sugar-based carbohydrates
(c) optional amphoteric and/or zwitterionic surfactants
(d) optional other auxiliaries or additives, including salts, and
(e) water,
with the provisos that the concentrates are free from anionic surfactants and contain hydrogenated castor oil a) in a quantity above 7,0 to 20 wt% and wherein the sum of quantities of (a) to (d) is in the range from 30% to 80% by weight and add up to 100% by weight by water.

It has surprisingly been found that a stabilizer concentrate containing hydrogenated castor oil in special amounts and in combination with special nonionic emulsifiers leads to concentrates with a high content of actives as well as a very low viscosity in case they are free of anionic surfactants. Additionally, the stabilizer concentrates according to the invention are very stable themselves and improve very well the stability of the wax in the personal care compositions, especially containing decomposition agents.

Especially, waxes, which are used in form of a dispersion as a pearlizer and/or opacifier in personal care compositions could be improved e.g. in their storage stability. Therefore, the pearlizing/opacifying white colour maintained and no separation of the personal care products was observed during storage. Surprisingly the effects were also observed for personal care compositions containing cationic polymers.

According to the invention the term "stabilizer concentrate(s)" or "concentrate(s)" are compositions with a high content of ingredients a) to d) and a reduced water content compared to common dispersions. According to the invention the sum of quantities of (a) to (d) is in the range from 30% to 80% by weight and add up to 100% by weight by water.

The abbreviation "wt%" means "weight percentage" and is synonym to "% by weight". According to the invention all weight percentages means the active matter weight percentage unless other specified.

According to the invention "waxes" are compounds having a melting point above 25°C, preferred above 50°C and especially above 80°C. The melting point was determined according to ISO 6321.

According to the invention the term "free from anionic surfactants" means that no anionic surfactants are added intentionally as a component or ingredient to prepare the concentrate; therefore, theoretically the concentrate comprises 0 wt% of anionic surfactants; in practice, the components of the concentrates may contain by-products, which may also be anionic surfactants. But these potential by-products are not added intentionally and therefore the concentrate is free from anionic surfactants according to the invention.

According to the invention the term "stabilizer" means that the concentrates has a stabilizing effect for waxes, which are in form of a dispersion contained in personal care compositions. The stabilizing effect of the concentrates was measured as storage stability (for at least 4 weeks at 40 °C) in view of the visual appearance of personal care compositions after storage. A stabilizing effect means that the visual appearance is unchanged, e.g. no separation or crystals should be observed, when the inventive concentrates are added.

### Concentrates

The concentrates according to the invention contain

### a) Hydrogenated castor oil (HCO)

Castor oil (CAS-Nr. 8001-79-4) is a vegetable oil and contains glycerides, especially triglycerides of fatty acids having C10 to C22 alkyl or alkenyl moieties which incorporate a hydroxyl group. Hydrogenation of castor oil produces hydrogenated castor oil by converting double bonds, which are present in the starting oil as ricinoleyl moieties. These moieties are converted to ricinoleyl moieties, which are saturated hydroxyalkyl moieties, e.g. hydroxystearyl.

The hydrogenated castor oil (HCO) may be processed in any suitable starting form, including, but not limited those selected from solid, molten and mixtures thereof. Useful hydrogenated castor oil (HCO) may have the following characteristics: more than 80 wt%, especially 80-90 wt% triglycerides of ricinoleic acid. The residue may be free fatty acids, water and other impurities.

Preferred is hydrogenated castor oil (HCO) with a melting point of from about 40 °C to about 100 °C, alternatively from about 65 °C to about 95 °C, and especially with Iodine value ranges of from about 0 to about 5, alternatively from about 0 to about 4, and alternatively from about 0 to about 2.6. The melting point of hydrogenated castor oil (HCO) can measured by using DSC: Differential Scanning Calorimetry.

Suitable hydrogenated castor oil (HCO) include those that are commercially available. Non-limiting examples of commercially available hydrogenated castor oil (HCO) suitable for use include: THIXCIN-R (supplied by Elementis) and Cutina^{®} HR (supplied by BASF Personal Care and Nutrition GmbH), both are supplied as a powder.

According to the invention the concentrates contain (a) hydrogenated castor oil in a quantity range from above 7.0- 20 wt%, preferred from 7.5 to 15 wt% and especially from 8.5 to 12 % by weight - based on concentrates.

### b) Nonionic emulsifiers

According to the invention the concentrates contain nonionic emulsifiers selected from the group consisting of fatty alcohol polyglycol ether, ethoxylated fatty acid glycerol ester; mixed ethers or mixed formals; polysorbates and sugar-based carbohydrates.

In context of the present invention, the term "nonionic surfactant" or "nonionic emulsifiers "are used synonymous; and it is also understood to mean a mixture of one or more nonionic emulsifiers.

If the nonionic emulsifiers contain polyglycol ether chains, these may have a conventional homolog distribution, but preferably have a narrow homolog distribution.

Examples for fatty alcohol polyglycol ethers are alkoxylation, especially ethoxylation and/or propoxylation products of fatty alcohols, especially linear fatty alcohols containing 6 to 22 carbon atoms. Preferred are adducts of linear, saturated fatty alcohols with 12 and/or 14 carbon atoms. Especially preferred are fatty alcohol adducts of 1- 7 mol Ethylene oxid and opt. 2-8 mol Propylene oxid. Suitable are the commercial products like Arlypon^{®} F-T, available by BASF Personal Care Nutrition GmbH.

Examples for ethoxylated fatty acid glycerol ester are - with the provisio that (b) is different from (a):
- partial esters of glycerol with unsaturated, linear or saturated, linear or branched fatty acids containing 6 to 22 carbon atoms and/or hydroxycarboxylic acids containing 3 to 18 carbon atoms and adducts thereof with 1 to 30 mol ethylene oxide;
- triglycerids, i.e. triesters of glycerol with unsaturated, linear or saturated, linear or branched fatty acids containing 6 to 22 carbon atoms and/or hydroxycarboxylic acids containing 3 to 18 carbon atoms and adducts thereof with 1 to 60 mol ethylene oxide;

Typical examples of suitable ethoxylated partial glycerides are addition products of 1 to 30 and preferably 5 to 10 mol ethylene oxide and glycerol partial esterfied with the mentioned fatty acids. Preferred examples hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process.

Within the ethoxylated fatty acid glycerol esters preference is given to a partial ester of ethoxylated glycerol with a fatty acid mixture derived from coco nut, in particular a mixture of partial esters of ethoxylated glycerol with a fatty acid mixture derived from coco nut, wherein the monoester content in the mixture of mono, di- and triester is more than 40% by weight. Suitable is the commercial product Cetiol^{®} HE, available by BASF Personal Care Nutrition GmbH.

Typical examples for ethoxylated triglycerides are addition products of 1 to 15 or 15 to 60 mol ethylene oxide with castor oil and/or hydrogenated castor oil. Suitable is the commercial product Eumulgin^{®} HRE 40 available by BASF Personal Care Nutrition GmbH.

Typical examples of polysorbates are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Examples for mixed ethers or formals are triblockcopolymers containing mono or polymers of Ethylenoxid and polymers of Propylenoxid in three blocks e.g. Pluracare^{®} L44 (Poloxamer124), Pluracare^{®} F 127 (Poloxamer 407).

Examples for sugar-based carbohydrates are alk(en)yl polyglycosides or glucuronic acid derivates and fatty acid N-alkylglucamides.

Examples for glucuronic acid derivates and fatty acid N-alkylglucamides are N-alkylglucamides derived fromlauric acid, myristic acid, palmitic acid, palmoleic acid, stearic acid, isostearic acid, oleic acid, elaidic acid, petroselinic acid, linoleic acid, linolenic acid and their technical mixtures. Particular preference is given to the use of a N-methyl glucamides and especially on the basis of a technical C₁₂ - C₁₄ - coconut fatty acid fraction. Suitable products are Glucopure^{®}Foam or Glucopure^{®}Sense obtainable by Clariant.

Examples for sugar-based carbohydrates are alkyl polyglycosides.

Alkyl polyglycosides are known nonionic surfactants which have in particular the formula (I),

RO-[G]ₚ (I)

in which
- R is an alkyl radical having 6 to 22 carbon atoms,
- G is a sugar radical having five or six carbon atoms and
- p is a number from 1 to 10.

They can be obtained by the relevant methods of preparative organic chemistry. The alkyl polyglycosides can be derived from aldoses or ketoses having 5 or 6 carbon atoms, preferably from glucose. The preferred alkyl polyglycosides are therefore alkyl polyglucosides. The index number p in the general formula (I) specifies the degree of polymerization (DP), i.e. the distribution of mono- and polyglycosides, and is a number between 1 and 10. Whereas p in a given compound must always be an integer and can here in particular assume the values p = 1 to 6, the value p for a particular alkyl polyglycoside is an analytically determined calculated parameter which in most cases is a fraction. Preferably, alkyl polyglycosides are used with an average degree of polymerization p of 1.1 to 3.0. Preference is given to those alkyl polyglycosides, from a technical applications point of view, for which the degree of polymerization is less than 1.7 and is particularly between 1.2 and 1.7.

The alkyl radical R can be derived from primary alcohols having 6 to 22, preferably 6 to 18 carbon atoms. Typical examples are caproic alcohol, caprylic alcohol, capric alcohol, decyl alcohol and undecyl alcohol, and also their technical grade mixtures, as obtained, for example, in the hydrogenation of technical grade fatty acid methyl esters or during the hydrogenation of aldehydes from Roelen's oxo synthesis. The alkyl radical R⁷ can also be derived from lauryl alcohol, myristyl alcohol, cetyl alcohol, palmoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol, brassidyl alcohol and also technical grade mixtures thereof.

In the context of the present invention, preference is given in particular to mixtures of different alkyl polyglycosides of the formula (I), in which R is derived from primary alcohol mixtures. According to one preference R is derived from primary alcohol mixtures comprising 10 to 50% by weight 8 and 10 carbon atoms and 50 to 90% by weight 12 to 16 carbon atoms.

According to another preference R is derived from primary alcohol mixtures comprising 75 to 95% by weight primary higher alcohols with 10 to 22 carbon atoms, especially derivated from fatty acid mixture obtained from coco nut, preferably 12 to 16 carbon atoms.

Suitable products are Plantacare^{®} 2000 and Plantacare^{®} 818, both available by BASF Personal Care Nutrition GmbH.

The concentrates preferable contain as nonionic emulsifiers (b) sugar-based carbohydrates and more preferred alk(en)yl polyglycosides, glucuronic acid derivates and fatty acid N-alkylglucamides and especially alk(en)ylpoly glyocosides.

Preferably the concentrates contain the nonionic emulsifiers (b) in the quantity range of from 10 to 60% by weight, preferred 20 to 40 and especially preferred 25 to 35 % by weight - based on concentrates.

Concentrates are preferred with a quantity ratio of nonionic emulsifiers b) : hydrogenated castor oil a) in the range from 2:1 to 4:1 , especially about 3:1 .

### c) Amphoteric and/or zwitterionic surfactants

Concentrates according to the invention may contain amphoteric and/or zwitterionic surfactants, especially amphoacetates and/or betaines.

Betaines are known surfactants which are mainly produced by carboxyalkylation, preferably carboxymethylation, of aminic compounds. The starting materials are preferably condensed with halocarboxylic acids or salts thereof, more particularly with sodium chloroacetate. Examples of suitable betaines are the carboxyalkylation products of secondary and, in particular, tertiary amines corresponding to formula (II): 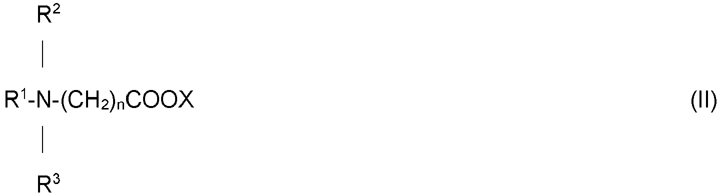 in which R¹ stands for alkyl and/or alkenyl groups containing 6 to 22 carbon atoms, R² stands for hydrogen or alkyl groups containing 1 to 4 carbon atoms, R³ stands for alkyl groups containing 1 to 4 carbon atoms, n is a number of 1 to 6 and X is an alkali metal and/or alkaline earth metal or ammonium. Typical examples are the carboxymethylation products of hexyl methyl amine, hexyl dimethyl amine, octyl dimethyl amine, decyl dimethyl amine, dodecyl methyl amine, dodecyl dimethyl amine, dodecyl ethyl methyl amine, C_{12/14} cocoalkyl dimethyl amine, myristyl dimethyl amine, cetyl dimethyl amine, stearyl dimethyl amine, stearyl ethyl methyl amine, oleyl dimethyl amine, C_{16/18} tallow alkyl dimethyl amine and technical mixtures thereof.

Other suitable betaines are carboxyalkylation products of amidoamines corresponding to formula (III): 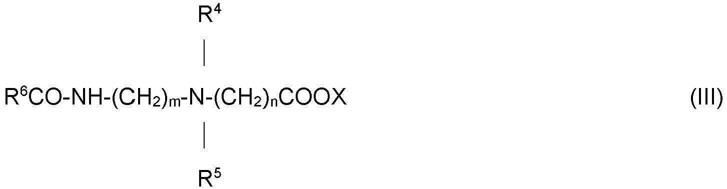 in which R⁶CO is an aliphatic acyl group containing 6 to 22 carbon atoms and 0 or 1 to 3 double bonds, m is a number of 1 to 3, R⁴ represents hydrogen or C₁₋₄ alkyl groups, R⁵ represents C₁₋₄ alkyl groups, n is a number of 1 to 6 and X is an alkali metal and/or alkaline earth metal or ammonium. Typical examples are reaction products of fatty acids containing 6 to 22 carbon atoms, namely caproic acid, caprylic acid, capric acid, lauric acid, myristic acid, palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid and erucic acid and technical mixtures thereof, with N,N-dimethyl aminoethyl amine, N,N-dimethyl aminopropyl amine, N,N-diethyl aminoethyl amine and N,N-diethyl aminopropyl amine which are condensed with sodium chloroacetate. It is preferred to use a condensation product of C_{8/18} cocofatty acid-N,N-dimethyl aminopropyl amide with sodium chloroacetate known under the CTFA name of *Cocamidopropyl Betaine.* Betaines distinguished by high purity are particularly preferred; in other words, low-salt betaines with a maximum salt content of 13% by weight, preferably 11% by weight and more particularly 7% by weight - based on active substance - are used. The corresponding salt is dependent on the production of the amphoteric surfactant; in the most common case, it is sodium chloride. In a particularly preferred embodiment, these betaines also have a low content of free fatty acids of at most 4% by weight and preferably at most 3% by weight, based on active substance.

Furthermore, imidazolinium betaines are also included. These substances are also known substances which can be obtained for example by cyclizing condensation of 1 or 2 mol of fatty acid with polyfunctional amines such as, for example, aminoethylethanolamine (AEEA) or diethylenetriamine. The corresponding carboxyalkylation products are mixtures of different open-chain betaines. Typical examples are condensation products of the abovementioned fatty acids with AEEA, preferably imidazolines based on lauric acid or again C_{12/14}-coconut fatty acid which are subsequently betainized with sodium chloroacetate.

Suitable Cocamidopropyl Betaine are commercially available like Dehyton^{®} PK 45 (supplied by BASF Personal Care and Nutrition GmbH).

In case the concentrates contain surfactants (c) it is preferred that they are selected from the group containing amphoteric and/or zwitterionic surfactants, especially betaines and/or amphoacetats.

The concentrates may contain said surfactants c) in a quantity range from 0 to 30 wt.% by weight - based on concentrates.

In case the concentrates contain surfactants (c) it is preferred to have a quantity ratio of nonionic emulsifiers (b) to amphoteric and/or zwitterionic surfactants (c) in the range of from 10:1 to 20:1.

### Optional other auxiliaries or additives, including salts d)

According to the invention the concentrates may contain d) other auxiliaries or additives, including salts.

Dependent on the production of the betain surfactant (c) salts may be contained as a by-product; in the most common cases, it is sodium chloride. Additionally, a low content of free fatty acids may be a possible by-product.

Examples for other auxiliaries are thickeners, complexing agents, nonaqueous solvents, preservatives and/or pH-adjusters. It is preferred not to add any auxiliaries, but it is still possible.

Preferred concentrates contain other auxiliaries or additives, including salts, (d) in a quantity range of from 0.1 to 15% by weight, based on concentrates substance.

According to one preferred embodiment of the invention the concentrates consist of
(a) above 7.0 to 20% by weight of hydrogenated castor oil,
(b) 10.0 to 60.0% by weight of nonionic emulsifiers,
(d) 0.1 to 15 % by weight of other auxiliaries or additives, including salts, and
(e) add up to 100% by weight by water.

According to another preferred embodiment of the invention the concentrates consist of
(a) 7.5 to 15.0% by weight of hydrogenated castor oil,
(b) 20.0 to 50.0% by weight of nonionic emulsifiers,
(d) 0.1 to 15 % by weight of other auxiliaries or additives, including salts, and
(e) add up to 100% by weight by water.

Both embodiments are preferred concentrates covered by at least claim 1, i.e. with the provisos that the concentrates are free from anionic surfactants and wherein the sum of quantities of (a) to (d) is in the range from 30% to 80% by weight and add up to 100% by weight by water. Especially preferred are concentrates wherein the sum of quantities of (a) to (d) is in the range from 40% to 80% by weight and add up to 100% by weight by water.

According to the invention the concentrates are flowable and have a viscosity (Brookfield, RVT; spindle 4; 10 rpm; 20 °C) preferred in the range of from 2.500 to 8.000 mPas, especially 3.000 to 7.000 mPas and more preferred between 3.000 and 5.500 mPas.

According to a further subject of the invention the concentrates are prepared by mixing components (a), (b) and optionally (c) and/or optionally (d) together, adding up with the necessary quantity of water and heating the mixture to about 85 to 95 °C before cooling down with agitation. It is preferred to heat the mixture to about 88-93 °C.

It is preferred to cool the reaction mixture down with agitation, for example with stirring, especially at least 5 to 150 minutes and preferred with 50-150 rpm. In the sense of the invention it is desired to avoid crystallization or recrystallization of the hydrogenated castor oil (a) during the process. For cooling a cooling rate of 15-30 °C per hour is preferred during the process.

### Wax dispersion

According to the invention the stabilizer concentrates are used for waxes selected from the group consisting of mono- and/or diesters of mono- and/or diethylene glycol, whereby the waxes are in form of a dispersion comprised in personal care compositions.

According to the invention the term "mono- and/or diesters of mono- and/or diesters of diethylene glycol" means monoesters of mono ethylene glycol, monoesters of diethylene glycol and diesters of diethylene glycol and their mixtures.

The following formula describes suitable esters of ethylene glycol:

R⁸CO(OA)_{q}OR⁷ (IV)

in which R⁸CO is a linear or branched, saturated or unsaturated acyl group containing 6 to 22 carbon atoms, R⁷ is hydrogen or has the same meaning as R⁸CO and A is a linear alkylene group containing 2 carbon atoms and q is a number of 2, provided that they are waxes at room temperature. Typical examples are monoesters of mono ethylene glycol and mono- and/or diesters of diethylene glycol (q=2) with fatty acids containing 16 to 22 and preferably 16 to 18 carbon atoms, such as palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid and erucic acid and technical mixtures thereof.

Preferred are mono- and/or diesters of diethylene glycol, especially with fatty acids with 6-22 carbon atoms.

According to one embodiment of the invention the waxes comprise mono- and/or diesters of diethylene glycol with a fatty acid mixture containing 85 to 100 wt% stearic acid and 0 to 15 wt% different further fatty acids with 12 to 22 carbon atoms - based on the total amount of fatty acids. The fatty acid mixture preferably contains 90 to 98 wt% stearic acid and 2 to 10 wt% other saturated fatty acids with 16 to 22 carbon atoms - based on the total amount of fatty acids - and in particular the fatty acid mixture consists of 90 to 96 wt% stearic acid and 4 to 10 wt% other saturated fatty acids with 16 to 22 carbon atoms. Other saturated fatty acids with 16 to 22 carbon atoms are palmitic acid, arachidic acid and behenic acid.

Preferably the wax comprises diethylene glycol fatty acid esters, especially diethylene glycol di fatty acid esters which, for technical reasons, contain 90 to 100 wt% diethylene glycol di fatty acid esters and 0 to 10% by weight of diethylene glycol mono fatty acid esters.

In a very particularly preferred embodiment the concentrates according to the invention stabili-ze wax dispersions, wherein the wax comprises 90 to 100 wt% diethylene glycol di fatty acid ester and 0 to 10 wt % diethylene glycol mono fatty acid ester - wt% based on the wax, and particularly preferred ester of a fatty acid mixture, which contains 90 to 98 wt% stearic acid and 2 to 10 wt% other saturated fatty acids with 16 to 22 carbon atoms - wt% based on the fatty acid mixture.

In a preferred embodiment the wax in the dispersions have an average particle diameter d50 in the range of 0.8 to 3.5 µm, especially in the range of 1.0 to 3.0 µm, wherein the distribution of the particle diameters shows preferable more than 50% are below 3 µm and 90% of all particles are below 7 µm. In particular, the wax particle have predominantly spherical particle shapes, especially 70% of all particles having a three-dimensional structure which is characterized in particular by a ratio of height: width: length of 1: 1: 1. Such particle shapes and particle quantities can be determined by laser diffraction with the Mastersizer^{®} 2000 device and the corresponding product description of MALVERN INSTRUMENTS GmbH, Marie-Curie-Straße 4/1, 71083 Herrenberg, Germany.

Preferably the wax dispersions contain the wax in quantities of 15 to 35 wt%, based on the wax dispersion.

In a further embodiment the wax dispersions contain additional surfactants and optional auxiliaries or additives. Additional surfactants can be nonionic surfactants, anionic surfactants and/or betaines.

Suitable nonionic surfactants may be the same or different as described as nonionic emulsifier b). Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, fatty acid partial glycerides, mixed ethers and mixed formals, alk(en)yl oligoglycosides, partially oxidized alk(en)yl oligoglycosides and glucuronic acid derivatives, fatty acid N-alkylglucamides, polyol fatty acid esters, sugar esters, sorbitan esters, polysorbates and amine oxides. If the nonionic surfactants contain polyglycol ether chains, these may have a conventional homolog distribution, but preferably have a narrowed homolog distribution.

Preferred nonionic surfactants in the wax dispersions are alk(en)yl oligoglycosides and/or fatty acid partial glycerides. In this context fatty acid partial glycerides means monoglycerides, diglycerides and their technical mixtures, which may still contain small amounts of glycerol and triglycerides due to the manufacturing process. Preferred fatty acid partial glycerides are technical mixtures of fatty acid partial glycerides which have a monoglyceride content in the range of 50 to 95 wt%, preferably 60 to 90 wt%, and are hereinafter also referred to as glycerol mono fatty acid esters. Preferred fatty acid partial glycerides are selected from the group formed by the glycerol mono-fatty acid ester, with lauric acid, isotridecanoic acid, myristinic acid, palmitic acid, palmoleic acid, stearic acid, isostearic acid, oleic acid, elaidic acid, petroselinic acid, linol acid, linolenic acid, elaeostearic acid, arachinic acid, gadoleic acid, behenic acid and erucic acid as well as their technical mixtures. Particularly preferred are mono fatty acid esters of (technical) glycerol and of fatty acids having 12 to 18 C atoms, preferably of a fatty acid mixture which contains 90 to 100% by weight of oleic acid - based on fatty acid mixture.

A suitable technical product on the market, for example, is Monomuls^{®} 90-O 18, a retail product of BASF Personal Care & Nutrition GmbH.

The fatty acid partial glycerides are preferably contained in quantities of 0.5 to 3.0, preferably 1.0 to 2 % by weight, based on the wax dispersion.

The wax dispersions may additionally contain alk(en)yl oligoglycosides as nonionic surfactants, especially in combination with fatty acid partial glycerides. Suitable alk(en)yl oligoglycosides were already described as nonionic emulsifier b) above. Preference is given to Plantacare^{®}818, a product obtainable by BASF Personal Care & Nutrition GmbH.

The wax dispersions may additionally contain anionic surfactants selected from the group consisting of fatty alcohol (ether) sulfates.

In particular, fatty alcohol (ether) sulfates of the general formula (V),

R'O (CH₂CH₂O)₀₋₁₀SO₃Y (V)

in which R' is a linear or branched alkyl and/or alkenyl radical having 6 to 22 carbon atoms and Y is an alkali metal and/or alkaline earth metal, ammonium, alkylammonium, alkanolammonium or glucammonium. If the number is zero (=0), this is fatty alcohol sulphate. Fatty alcohol ether sulphates (number from 1 to 10) are preferred. Typical examples are the sulphates of investment products of on average 1 to 10 and in particular 2 to 5 mol ethylene oxide of capron alcohol, capryl alcohol, 2-ethylhexyl alcohol, capri alcohol, lauryl alcohol, isotridecyl alcohol, myristyl alcohol, cetyl alcohol, Palmoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and their technical mixtures in the form of their sodium and/or magnesium salts. The fatty alcohol (ether) sulfates may have both a conventional and a narrowed homologue distribution. It is particularly preferred to use fatty alcohol ether sulfates based on adducts of, on average, 1 to 6 mol and preferably 1 to 3 mol ethylene oxide with lauryl ether sulfate, preferably with technical C_{12/14}, or C_{12/18} coconut fatty alcohol fractions in the form of their sodium and/or magnesium salts.

A suitable technical product on the market, for example, is Texapon^{®} N70, a retail product of BASF Personal Care & Nutrition GmbH.

Preferred wax dispersions contain anionic surfactants, especially lauryl ether sulphate with an average degree of ethoxylation of 1 or 2, preferably in an amount of 8 to 15, preferably 9 to 12 wt% - based on wax dispersion.

Additionally the wax dispersions may contain betaine surfactants, which have been already described as component c) of the inventive concentrate, preferably betaine surfactants according to formula (I) and/or (II) and in particular according to formula (II), and within this group it is preferred to use a condensation product of C_{8/18} cocofatty acid-N,N-dimethyl aminopropyl amide with sodium chloroacetate known under the CTFA name of Cocamidopropyl Betaine.

Preferred wax dispersions contain betaine surfactants, especially Cocamidopropyl Betaine, preferably in an amount of 0 to 5, preferably 0.5 to 2.5 wt% - based on wax dispersion.

It may possible that the dispersions contain additionally in an amount from 0 to 20 wt% auxiliaries or additives, including salts, which have been for example described as component d) in the concentrates, preferably in an amount of 0 to 10 wt%.

In the end the wax dispersions contain up to 100 wt% water.

According to one embodiment of the invention it is preferred that the concentrates stabilize waxes in personal care compositions, whereby the wax is used in form of a wax dispersion comprising - wt% based on the wax dispersion -
20 to 35 wt%, preferably 25 to 30 wt%, waxes selected from the group consisting of mono- and/or diesters of mono- and/or diethylene glycol, and
0.5 to 3.0 wt%, preferably 1.0 to 2 wt%, nonionic surfactants and
8.0 to 15.0 wt%, preferably 9.0 to 12.0 wt%, anionic surfactants and
0 to 5.0 wt%, preferably 0.5 to 2.5 wt%, betaine surfactants and
0 to 10 wt% auxiliaries or additives, including salts, and
up to 100 wt% water.

In particular, consisting of
20 to 35 wt%, preferably 25 to 30 wt%, waxes selected from diester of diethylene glycol fatty acid ester with 90 to 100 wt% diethylene glycol difatty acid content and 90 to 98 wt% stearic acid and
2 to 10 wt% other saturated fatty acids with 16 to 22 carbon atoms content in the fatty acid mixture, and
0.5 to 3.0 wt%, preferably 1.0 to 2 wt%, fatty acid partial glycerides and
8.0 to 15.0 wt%, preferably 9.0 to 12.0 wt%, lauryl ether sulphate with an average degree of ethoxylation of 1 or 2 and
0 to 5.0 wt%, preferably 0.5 to 2.5 wt%, Cocamidopropyl Betaine, and
0 to 10 wt% auxiliaries or additives, including salts, and
up to 100 wt% water.

According to a second embodiment of the invention the wax dispersions do not contain anionic surfactants selected from the group consisting of sulphate and/or ether sulphate, especially do not contain any anionic surfactants. Within this embodiment of the invention the wax dispersions are comprising- wt% based on the wax dispersion-
15 to 25 wt%, waxes selected from the group consisting of mono- and/or diesters of mono- and/or diethylene glycol, and
0.5 to 5.0 wt%, preferably 1.0 to 3 wt%, fatty acid partial glycerides and
10 to 20 wt%, nonionic surfactants selected from the group of alk(en)yl oligoglycosides and
0 to 10 wt% auxiliaries or additives, including salts, and
up to 100 wt% water.

In particular, consisting of
15 to 25 wt%, waxes selected from diester of diethylene glycol fatty acid ester with 90 to 100 wt% diethylene glycol difatty acid content and 90 to 98 wt% stearic acid and 2 to 10 wt% other saturated fatty acids with 16 to 22 carbon atoms content in the fatty acid mixture, and
0.5 to 5.0 wt%, preferably 1.0 to 3 wt%, fatty acid partial glycerides and
10 to 20.0 wt% nonionic surfactants, especially alk(en)yl oligoglycosides and
0 to 10 wt% auxiliaries or additives, including salts, and
up to 100 wt% water.

According to the invention the concentrates stabilize the wax in the personal care compositions, whereby the wax is in the form of the above described wax dispersions very good, especially personal care compositions comprising additionally cationic polymers show good results as the depositing agents do not accelerate any separation of the waxes.

Therefore, a further subject of the invention is the use of concentrates as claimed as a stabilizer for waxes selected from the group consisting of mono- and/or diesters of mono- and/or diethylene glycol, whereby the waxes are in form of a dispersion comprised in personal care compositions.

It is preferred to use the concentrates as claimed as a stabilizer for waxes in form of a wax dispersion, which are used as a pearlizing and/or opacifier agent in personal care compositions, preferable in personal care compositions comprising cationic polymers.

And a further subject of the invention is the use of concentrates as claimed, wherein the concentrates are produced by mixing components (a), (b) and optionally (c) and/or optionally (d) together, adding up with the necessary quantity of water, heating the mixture to about 85 to 95 °C before cooling down with agitation.

It is preferred to use the concentrates in a quantity range from 0.1 to 5 wt % - preferred 0.5-1.5 wt% - calculated as active matter and with respect to personal care composition.

The use of the concentrates effects a significantly higher storage stability, i.e. after weeks no optical changes of the personal compositions could be observed, ie. no streaks, no crystals or separation of the wax occurred.

### Personal care compositions

Personal care compositions are to be understood here as all compositions known to a person skilled in the art which are exclusively or primarily intended to be applied externally to the human body or hair for the cleaning, caring, protection, and maintaining of a good condition, perfuming, changing the appearance or for influencing. Preferably the concentrates are used in liquid personal care compositions, in particular surface-active personal care compositions, such as, for example, foam baths, shower gels, shower baths, shower milks, shower creams, shampoos, hair masks, hair milks and hair conditioners.

Another subject according to the invention are personal care comprising
A) 0.1wt% to 5 wt%, especially 0.25 wt% to 3 wt% of the concentrates as claimed in at least one of the claims 1 to 13 and
B) wax dispersions as already described, comprising waxes selected from the group consisting of mono- and/or diesters of mono- and/or diethylene glycol and
C) one or more detersive surfactants selected from the groups selected from anionic surfactants, nonionic surfactants, amphoteric surfactants and/or zwitterionic surfactants and
D) optional cationic polymers and
E) optional other component(s) different from C) and/or D).

Preferably the personal care compositions - based on weight % in personal care composition - comprising
A) 0.1 wt% to 5.0 wt%, especially 0.25 wt% to 3 wt % of the concentrates as claimed in at least one of the claims 1 to 13,
B) 0.1 wt% to 5 wt%, especially 0.5 wt% to 2.5 wt% of the wax dispersions already described, comprising waxes selected from the group consisting of mono- and/or diesters of mono- and/or diethylene glycol and
C) 1.0 to 30 wt%, especially 5.0 to 25.0 wt% one or more detersive surfactants selected from the groups selected anionic surfactants, nonionic surfactants, amphoteric and/or zwitterionic surfactants,
D) 0 to 5 wt%, especially 0.01 to 1 wt% cationic polymers,
E) up to 100 wt% other components, different from C) and/or D).

### C) Anionic surfactants, nonionic surfactants, amphoteric and/or zwitterionic surfactants

Examples of anionic surfactants are soaps, alkyl benzenesulfonates, alkane sulfonates, olefin sulfonates, alkyl ether sulfonates, glycerol ether sulfonates, α-methyl ester sulfonates, sulfo fatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, the polyglycol ether chains may have a conventional homolog distribution, although they preferably have a narrow homolog distribution. Particularly suitable anionic surfactants in the preparations according to the invention are alkyl ether sulfates, which have already been disclosed in formula (V).

Alkyl ether sulfates ("ether sulfates") are known anionic surfactants which, on an industrial scale, are produced by SO₃ or chlorosulfonic acid (CSA) sulfation of fatty alcohol or oxoalcohol polyglycol ethers and subsequent neutralization. The ether sulfates may have both a conventional homolog distribution and a narrow homolog distribution. It is particularly preferred to use ether sulfates based on adducts of, on average, 1 to 6 mol and preferably 1 to 3 mol ethylene oxide with technical C_{12/14} or C_{12/18} coconut fatty alcohol fractions in the form of their sodium and/or magnesium salts.

Further anionic surfactants useful within the context of the present invention are alpha-sulfo fatty acid di salts according to the formular (VI) specified above

R⁹CH(SO₃M1)COOM2 (VI),

in which the radical R⁹ is a linear or branched alkyl or alkenyl radical having 6 to 18 carbon atoms and the radicals M1 and M2 - independently of one another - are selected from the group comprising H, Li, Na, K, Ca/2, Mg/2, ammonium and alkanolamines. In this connection, particularly preferred alkanolamines are monoethanolamine, diethanolamine, triethanolamine and monoisopropanolam ine.

In a preferred embodiment, the radical R⁹ in the formula (VI) is a saturated, linear alkyl radical having 10 to 16 carbon atoms. The radicals M1 and M2 in formula (VI) are preferably selected from the group comprising H (hydrogen) and Na (sodium).

The compounds can be prepared by all methods known appropriately to those skilled in the art. A particularly preferred method of preparation here is the sulfation of the corresponding carboxylic acids. Here, the corresponding carboxylic acid and in particular the corresponding fatty acids are reacted with gaseous sulfur trioxide, the sulfur trioxide being used preferably in an amount such that the molar ratio of SO₃ to fatty acid is in the range from 1.0 : 1 to 1.1 : 1. The crude products obtained in this way, which are acidic sulfation products, are then partially or completely neutralized, preference being given to complete neutralization with aqueous NaOH. If desired, it is also possible to undertake purification steps and/or a bleaching (for adjusting the desired pale color of the products).

Preferred alpha-sulfo fatty acid di salts are technical-grade mixtures of the alpha-sulfo fatty acid desalts, which is commercially available as Texapon SFA^{®} from BASF Personal Care Nutrition GmbH.

In addition, amphoteric and/or zwitterionic surfactants may be used in addition with or instead of the anionic surfactants. Suitable surfactants have already been described as component c) of the inventive concentrate, preferred are betaine surfactants according to formula (II) and/or (III) are useful and in particular the condensation product of C_{8/18} cocofatty acid-N, N-dimethyl aminopropyl amide with sodium chloroacetate known under the CTFA name of Cocamidopropyl Betaine is preferred.

In addition, nonionic surfactants may be used in addition with or instead of the anionic surfactants, amphoteric or zwitterionic surfactants. Suitable nonionic surfactants have already been described as component b) of the inventive concentrate, preferred are nonionic surfactants selected from the group consisting of fatty alcohol polyglycol ether, ethoxylated fatty acid glycerol ester; mixed ethers or mixed formals; polysorbates and sugar-based carbohydrates.

In particular alk(en)ylpolyglucosids as described according to formular (I) are preferred as nonionic surfactants and especially the already disclosed products Plantacare^{®} 2000 and Plantacare^{®} 818, both available by BASF Personal Care Nutrition GmbH.

It is preferred to use amounts from 1.0 to 30 wt%, especially 5.0 to 25.0 wt%, anionic surfactants, amphoteric and/or zwitterionic surfactants and/or nonionic surfactants - based calculated as active matter ad in respect to the personal care composition.

### D) Cationic polymers

Cationic polymers are known deposition agents, i.e. by using them in the personal care composition they deposit on skin and/or hair and give them a pleasant and a soft feeling. However, personal care compositions containing cationic polymers like cationic guar polymers have problems with their stability in case of presence of the wax dispersions as the wax(es) tend(s) to sediment more readily, probably a consequence of the deposition properties of the cationic polymers. This problem can be overcome by adding the concentrates according to the invention for stabilization the wax in the personal care composition.

Preferred personal care composition also comprises a cationic polymer. These cationic deposition polymers can include at least one of a cationic guar polymer, a cationic non-guar galactomannan polymer, a cationic tapioca polymer, a cationic copolymer of acrylamide monomers and cationic monomers, and/or a synthetic, non-crosslinked, cationic polymer. Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, Amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®}550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol.

Especially, the personal care composition may comprise cationic polymer selected from the group consisting of cationically modified cellulose derivatives, PQ 10, PQ 67, cationically modified guar derivatives, such as, for example, Dehyquart^{®} Guar N, guar hydroxypropyltrimonium chloride, cationic homo- or copolymers based on acrylamide, cationic homo- or copolymers based on vinyl pyrrolidone, cationic homo- or copolymers based on quaternized vinyl imidazole and cationic homo- or copolymers based on methacrylates.

In particular, cationically modified guar derivates, preferably Guar Hydroxypropyltrimonium Chloride, are present.

It is preferred to use amounts from 0 to 5 wt%, especially 0.01 to 1.0 wt% cationic polymersbased on the personal care composition.

### E) Other components

For an end-user application, the cosmetic formulations may comprise a series of further auxiliaries and additives, such as, for example, water, bodying agents, viscosity reducers, thickeners, salts, superfatting agents, stabilizers, polymers, fats, waxes, silicones, lecithins, protein hydrolyates, phospholipids, biogenic active ingredients, UV sunscreen factors, antioxidants, deodorants, antiperspirants, antidandruff agents, film formers, swelling agents, insect repellents, self-tanning agents, tyrosinase inhibitors (depigmenting agents), hydrotropes, solubilizers, preservatives, perfume oils, dyes, and the like, including water.

For reducing the viscosity, the personal care compositions may additionally contain polyols as an optional component. Suitable polyols preferably contain 2 to 15 carbon atoms and at least two hydroxyl groups. The polyols may contain other functional groups, more especially amino groups, or may be modified with nitrogen. Typical examples are
▪ glycerol;
▪ alkylene glycols such as, for example, ethylene glycol, diethylene glycol, propylene glycol, butylene glycol, hexylene glycol and polyethylene glycols with an average molecular weight of 100 to 1.000 dalton;
▪ technical oligoglycerol mixtures with a degree of self-condensation of 1.5 to 10 such as, for example, technical diglycerol mixtures with a diglycerol content of 40 to 50% by weight;
▪ methylol compounds such as, in particular, trimethylol ethane, trimethylol propane, trimethylol butane, pentaerythritol and dipentaerythritol;
▪ lower alkyl glucosides, particularly those containing 1 to 8 carbon atoms in the alkyl group, for example methyl and butyl glucoside;
▪ sugar alcohols containing 5 to 12 carbon atoms, for example sorbitol or mannitol;
▪ sugars containing 5 to 12 carbon atoms, for example glucose or sucrose;
▪ amino sugars, for example glucamine;
▪ dialcoholamines, such as diethanolamine or 2-aminopropane-1,3-diol.

The polyols are used in quantities of typically 0.1 to 10% by weight, preferably 0.5 to 5% by weight and more particularly 0.7 to 3% by weight, based on the personal care composition. If larger quantities of polyol, preferably glycerol or ethylene glycol, are used, the concentrates are simultaneously stabilized against microbial infestation.

Suitable oil components are, for example, Guerbet alcohols based on fatty alcohols containing 6 to 18 and preferably 8 to 10 carbon atoms, esters of linear C₆₋₂₂ fatty acids with linear C₆₋₂₂ fatty alcohols, esters of branched C₆₋₁₃ carboxylic acids with linear C₆₋₂₂ fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆₋₂₂ fatty acids with branched alcohols, more particularly 2-ethyl hexanol, esters of hydroxycarboxylic acids with linear or branched C₆₋₂₂ fatty alcohols, more especially Dioctyl Malate, esters of linear and/or branched fatty acids with polyhydric alcohols (for example propylene glycol, dimer diol or trimer triol) and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, more particularly benzoic acid, esters of C₂₋₁₂ dicarboxylic acids with linear or branched alcohols containing 1 to 22 carbon atoms or polyols containing 2 to 10 carbon atoms and 2 to 6 hydroxyl groups, vegetable oils, branched primary alcohols, substituted cyclohexanes, linear and branched C₆₋₂₂ fatty alcohol carbonates, Guerbet carbonates, esters of benzoic acid with linear and/or branched C₆₋₂₂ alcohols (for example Finsolv^{®} TN), linear or branched, symmetrical or nonsymmetrical dialkyl ethers containing 6 to 22 carbon atoms per alkyl group, ring opening products of epoxidized fatty acid esters with polyols, silicone oils and/or aliphatic or naphthenic hydrocarbons, for example squalane, squalene or dialkyl cyclohexanes.

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

Suitable thickeners are, for example, Aerosil types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates or alkyl oligoglucosides and electrolytes, such as sodium chloride and ammonium chloride.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. Typical examples of fats are glycerides while suitable waxes are inter alia natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

Salts like sodium chloride can be incorporated as a by-product.

In the context of the invention, biogenic agents are, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Additionally, film formers may be present. Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof and similar compounds.

If desired, further protein hydrolyzates known from the prior art may be used, for example based on keratin such as the commercially available Nutrilan^{®} Keratin W PP, or based on wheat, such as Gluadin^{®} WLM Benz, Gluadin^{®} WK or Gluadin^{®} WP. It is also possible to add small amounts of free amino acids such as lysine or arginine.

Cosmetic deodorants counteract, mask or eliminate body odors. Body odors are formed through the action of skin bacteria on apocrine perspiration which results in the formation of unpleasantsmelling degradation products. Accordingly, deodorants contain active principles which act as germ inhibitors, enzyme inhibitors, odor absorbers or odor maskers.

Basically, suitable germ inhibitors are any substances which act against gram-positive bacteria such as, for example, 4-hydroxybenzoic acidand salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), antibacterial perfumes, thymol, thyme oil, eugenol, nettle oil, menthol, mint oil, farnesol, phenoxyethanol, glycerol monolaurate (GML), diglycerol monocaprate (DMC), salicylic acid-N-alkylamides such as, for example, salicylic acid-n-octyl amide or salicylic acid-n-decyl amide.

Suitable enzyme inhibitors are, for example, esterase inhibitors. Esterase inhibitors are preferably trialkyl citrates, such as trimethyl citrate, tripropyl citrate, triisopropyl citrate, tributyl citrate and, in particular, triethyl citrate (Hydagen^{®} CAT, Henkel KGaA, Düsseldorf, FRG). Esterase inhibitors inhibit enzyme activity and thus reduce odor formation. Other esterase inhibitors are sterol sulfates or phosphates such as, for example, lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester, and zinc glycinate.

Suitable odor absorbers are substances which are capable of absorbing and largely retaining the odor-forming compounds. They reduce the partial pressure of the individual components and thus also reduce the rate at which they spread. An important requirement in this regard is that perfumes must remain unimpaired. Odor absorbers are not active against bacteria. They contain, for example, a complex zinc salt of ricinoleic acid or special perfumes of largely neutral odor known to the expert as "fixateurs" such as, for example, extracts of labdanum or styrax or certain abietic acid derivatives as their principal component. Odor maskers are perfumes or perfume oils which, besides their odor-masking function, impart their particular perfume note to the deodorants. Suitable perfume oils are, for example, mixtures of natural and synthetic fragrances. Natural fragrances include the extracts of blossoms, stems and leaves, fruits, fruit peel, roots, woods, herbs and grasses, needles and branches, resins and balsams. Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, p-tert.butyl cyclohexylacetate, linalyl acetate, phenyl ethyl acetate, linalyl benzoate, benzyl formate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Antiperspirants reduce perspiration and thus counteract underarm wetness and body odor by influencing the activity of the eccrine sweat glands. Aqueous or water-free antiperspirant formulations typically contain astringent active agents for example salts of aluminium, zirconium or zinc. Suitable antihydrotic agents of this type are, for example, aluminium chloride, aluminium chlorohydrate, aluminium dichlorohydrate, aluminium sesquichlorohydrate and complex compounds thereof, for example with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, for example with amino acids, such as glycine. Oil-soluble and water-soluble auxiliaries typically encountered in antiperspirants may also be present in relatively small amounts. Oil-soluble auxiliaries such as these include, for example, inflammation-inhibiting, skin-protecting or pleasant-smelling essential oils, synthetic skin-protecting agents and/or oil-soluble perfume oils.

Typical water-soluble additives are, for example, preservatives, water-soluble perfumes, pH regulators, for example buffer mixtures, water-soluble thickeners, for example water-soluble natural or synthetic polymers such as, for example, xanthan gum, hydroxyethyl cellulose, polyvinyl pyrrolidone or high molecular weight polyethylene oxides.

Suitable antidandruff agents are climbazol, octopirox and zinc pyrithione.

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

In addition, hydrotropes, for example ethanol, isopropyl alcohol or polyols, may be used to improve flow behavior.

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive"). Suitable insect repellents are N,N-diethyl-m-toluamide, pentane-1,2-diol or Ethyl Butylacetylaminopropionate. A suitable self-tanning agent is dihydroxyacetone.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Dyes which can be used are the substances approved and suitable for cosmetic purposes, as listed, for example, in the publication "Kosmetische Färbemittel" [Cosmetic Colorants] from the Farbstoffkommission der Deutschen Forschungsgemeinschaft [Dyes commission of the German research society], Verlag Chemie, Weinheim, 1984, pp. 81-106. Examples are cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). As a luminescent dye, it is also possible for luminol to be present. These dyes are usually used in concentrations of from 0.001 to 0.1% by weight, based on the total mixture.

It is preferred to use these other components E) in amounts up to 100 wt% - based on the personal care composition.

Finally, another subject of the invention is a process for producing personal care composition as claimed characterized in that one or more surfactants C) and the wax dispersion B) and optional cationic polymers D) are mixed at room temperature, and the concentrates A) as claimed in at least one of the claims 1 to 13 are added and stirred together, wherein the optionally other components E) can be added before or after the addition of concentrates A).

### Examples

### Example 1: Concentrates (= Phase A) with hydrogenated castor oil as stabilizers

The concentrates were prepared by mixing all compounds listed in the following table 1 in the amount as stated (% weight as active matter) at 90 °C until homogenous and cool down to room temperature (approx. 25 °C) with agitation (100 rpm) for 5-120 minutes.

The following ingredients were used (Active Matter = AS):
Dehyton^{®} PK 45: Cocoamidopropylbetain: with 37% by weight active matter; 7% by weight soda chloride and up to 100% by weight water
Cutina^{®} HR Powder: Hydrogenated Castor Oil; 100% by weight active matter
Cetiol^{®} HE: nonionic surfactant; mono- and diester of fatty acid mixture based on coconut oil and glycerol, ethoxylated with 7 mol ethylene oxid
Plantacare^{®} 2000: aqueous solution of C8-C16 fatty alcohol poly glycoside; INCI: Decyl Glucoside; nonionic surfactant; about 51-55% active matter
Plantacare^{®} 818: aqueous solution of C8-C18 fatty alcohol poly glycoside; INCI Coco-glucoside; nonionic surfactant; about 51-53% active matter
Texapon^{®} N70: aqueous solution of C12-C14 Alkylethersulfat Na-salt; INCI: Sodium Laureth Sulfate; anionic surfactant; about 70% active matter

**Table 1a**

| **Concentrate 1** | | | **1a** | **1b** | **Comparison Example C 1a** |
|---|---|---|---|---|---|
| **Phase A / Component** | **INCI/Ingredients** | **Product name** | %AS by weight | %AS by weight | %AS by weight |
| | Cocoamidopropyl Betaine (c) | Cocoamidopropyl Betaine via De-hyton^{®} PK 45 | - | - | 37 |
| | Hydrogenated Castor Oil (a) | Cutina^{®} HR Powder | 10 | 10 | 2.7 |
| | C8-C10- Polyglucoside (b) | Plantacare^{®} 2000 | 30 | - | - |
| | C8-C18 (Coco)-polyglucoside (b) | Plantacare^{®} 818 | - | 30 | - |
| | C12-C14 Alkylethersulfat Na-salt | Texapon^{®} N70 | - | - | 5 |
| | Benzoic acid (d) | | 0.45 | 0.45 | |
| | Citric acid (50%ig AM) (d) | | 1.00 | 1.00 | |
| | Sum (a) to (d) | | 41.45 | 41.45 | 45.3 |
| | Water (e) | | Up 100 | Up 100 | Up 100 |
| Weight Ratio (b): (a) | | | 3:1 | 3:1 | 11.5:1 |
| Viscosity (Brookfield; RVT; spindle 4; 10 rpm; in mPas 20 °C) /40°C | | | 3120/3560; flowable | 5120/4960; flowable | 145.000; sticky; not flowable |

Table 1a shows that the inventive concentrates with nonionic emulsifiers and hydrogenated Castor Oil (a) in amount above 7 wt% show a very low viscosity compared to a concentrate containing betaine and additional anionic surfactants (comparison example C1a).

**Table 1b Comparison Examples C1b-C1d)**

| | | | **C1b** | **C1c** | **C1d** |
|---|---|---|---|---|---|
| **Phase A / Component** | **INCI/Ingredients** | **Product name** | %AS by weight | %AS by weight | %AS by weight |
| | | | | | |
| | Hydrogenated Castor Oil (a) | Cutina^{®} HR Powder | 10 | 10 | 10 |
| | C8-C10- Polyglucoside (b) | Plantacare^{®} 2000 | 22.5 | - | 25 |
| | C8-C18 (Coco)-polyglucoside (b) | Plantacare^{®} 818 | - | 25 | - |
| | C12-C14 Alkylethersulfat Na-salt | Texapon^{®} N70 | 7.50 | 5 | 5 |
| | Benzoic acid (d) | | 0.45 | 0.45 | 0.45 |
| | Citric acid (50%ig AM) (d) | | 1.00 | 1.00 | 1.00 |
| | Sum (a) to (d) | | 41.45 | 41.45 | 41.45 |
| | Water (e) | | Up 100 | Up 100 | Up 100 |
| Weight Ratio (b): (a) | | | 3:1 | 3:1 | 11.5:1 |
| Viscosity (Brookfield; RVT; spindle 4; 10 rpm; in mPas 20 °C) | | | Solid/not measurable | pasty/not measurable | pasty/not measurable |
| Viscosity (Brookfield; RVT; spindle TE Helipath in mPas 40 °C) | | | Pasty/not measurable | 115000 | 81600 |

Table 1 b shows that the comparison concentrates with nonionic emulsifiers, hydrogenated Castor Oil (a) and anionic surfactants are solid or pasty, i.e show a very high viscosity compared to the inventive concentrates without anionic surfactants according to table 1a.

### Example 2: Shower creams

The concentrates according to Example 1 of the invention were used in shower creams containing wax dispersion WD-1 (% by weight-based on active matter; AS 40,8%):
27.0 wt% of a mixture of about 6 wt% of monoester and about 94 wt% of diester of ethylene glycol, wherein the ester building fatty acid had about
90-98.5 wt of C-18 atoms and 2-10 wt C16+C20+C22 carbon atoms (measured with GC) 10 wt% Sodium Laureth Sulfate (with 1 EO)
1.5 - 2.0 wt% Cocamidopropyl Betaine
1.0 - 3.0 wt% Glyceryl monooleate
up to 100 wt% water, citric acid and benzoate.

The dispersed wax had an average particle size d50 - measured via laser diffraction (Mastersizer 2000^{®}) between 1.5-2.5 µm.

Or wax dispersion WD-2 (% by weight-based on active matter; AS about 39%):
22 wt% of a mixture of about 6 wt% of monoester and about 94 wt% of diester of ethylene glycol, wherein the ester building fatty acid had about
90-98.5 wt of C-18 atoms and 2-10 wt C16+C20+C22 carbon atoms (measured with GC)
10-20 wt% Coco-Glucoside
1.55 wt% Glyceryl monooleate
up to 100 wt% water, citric acid and benzoate.

The dispersed wax had an average particle size d50 - measured via laser diffraction (Mastersizer 2000^{®}) between 1.5 - 3.0 µm.

Or wax dispersion WD-3 (% by weight-based on active matter; AS 40,8%):
34.0 wt% of a mixture of about 6 wt% of monoester and about 94 wt% of diester of ethylene glycol, wherein the ester building fatty acid had about
90-98.5 wt of C-18 atoms and 2-10 wt C16+C20+C22 carbon atoms (measured with GC)
10 wt% Sodium Laureth Sulfate (with 1 EO)
1.5 - 2.0 wt% Cocamidopropyl Betaine
up to 100 wt% water, citric acid and benzoate.

The wax dispersions were used as Phase B).

The following further ingredients were used:
Dehyquart^{®} Guar N is a guar gum, 2-hydroxy-3-(trimethylammonio)propylether, chloride; INCI:Guar Hydroxypropyltrimonium Chloride; AS about 90%
Plantacare^{®} 818: aqueous solution of C8-C18 fatty alcohol poly glycoside; INCI Coco-glucoside; nonionic surfactant; about 51-53% active matter
Texapon^{®} SB 3 KC: Di-sodium salt of Laurylpolyglycolether sulfosuccinate; INCI: Disodium Laureth Sulfosuccinate; anionic surfactants 31.5-34.5%
Jordapon^{®}SCI: Sodium Cocoyl Isethionate; AS:at least 82%
Arlypon^{®} TT: a mixture of tethoxylated and propxylated -Trimethylolpropantrioleate and ethoxylated fatty alcohol; INCI: PEG/PPG-120/10 Trimethylolpropane Trioleate (and) Laureth-2
Plantasil^{®} 4V: Mixture of ethoxylated hydrogenated castor oil, ethoxylated glycerylfatty acid esters, ethoxylated polyolester and glycerin; INCI: PEG-40 Hydrogenated Castor Oil (and) PEG-7 Glyceryl Cocoate (and) PEG/PPG- 120/10 Trimethylolpropan Trioleate (and) Glycerin
Cetiol^{®} LDO: Blend of Dicaprylyl Ether and Lauryl Alcohol
Covi-ox^{®} T 90 C: Tocopherol
Lamesoft^{®} PO 65: Coco-Glucoside (and) Glyceryloleate
D- Panthenol ^{®} 75 W: Panthenol
Lamesoft^{®} TM Benz: Glycol Distearate, Coco-Glucoside, Glyceryl Oleate, Glyceryl Stearate

The shower creams according Table 2 were prepared by the following process: all components of Phase B) and C) and D) were mixed together under stirring at room temperature (23 °C) until they were completely homogeneous. The concentrates prepared according to Example 1 (= Phase A) were added and then Phase E) was added and both stirred until a completely homogeneous shower cream product was obtained. The components were used in form of the above mentioned sales products and in the indicated quantities by weight; the active matter of the active ingredients is indicated as (%AS).

**Table 2: Shower Creams**

| **Example 2** | | | **2a** | **2b** | **2c** | **2d** | **2e** | **Comparison Example 2** |
|---|---|---|---|---|---|---|---|---|
| **Concentrate** | | | 1a | 1b | 1b | 1b | 1b | no |
| | **Product name** | **INCI/active Ingredient** | % by weight (%AS) | % by weight (%AS) | % by weight (%AS) | % by weight (%AS) | % by weight (%AS) | % by weight (%AS) |
| Phase B)+C)+D) | Water, demin. | Aqua; water demin. | 53.93 | 54.50 | 54.5 | 51.81 | 46.59 | 43.45 |
| | Citric Acid | Citric Acid (50% solut.) | 0.20 (AS 0.1) | 0.84 (AS 0.42) | - | - | - | 0.20 |
| | Dehyquart^{®} Guar N | Guar Hydroxypropyltrimonium Chloride | 0.20 (AS 0.18) | - | - | 0.2 (AS 0.18) | 0.3 (AS 0.27) | 0.20 |
| | Dehyton^{®} PK 45 | Cocoamidopropyl Betaine | - | 4.16 (AS 1.54) | 4.16 (AS 1.54) | 4.16 (AS 1.54) | 10.81 (AS 3.99) | 5.40 |
| | Sodium Benzoate | Sodium Benzoate | 0,50 | 0.50 | 0.50 | 0.50 | 0.50 | 0.50 |
| | Plantacare^{®} 818 UP | C8-C18 (Coco)-polyglucoside | - | - | - | - | 8.0 (AS 4.16) | |
| | Texapon^{®} N 70 | Sodium Laureth Sulfate | 14.30 | 14.30 (AS 10.01) | 14.30 (AS 10.01) | 14.30 (AS 10.01) | | 14.30 |
| | Texapon^{®} SB 3 KC | Disodium Laureth Sulfosucci-nate | | | | | 6.0(AS 2.0) | |
| | Glycerin | Glycerin | 2.00 | - | - | 2.00 | - | 2.00 |
| | Wax dispersion WD1 | See above | 2.20 | 2.20 | 2.20 (AS 0.89) | - | - | 2.20 |
| | Wax Dispersion WD2 | See above | - | - | - | 3.0 (AS 1.17) | 3.0 (AS 1.17) | |
| Phase A) | Concentrates see Table 1 | | 5.55 (AS 2.3) | 1.50 (AS 0.62) | 1.50 (AS 0.62) | 1.50 (AS 0.62) | 1.50 (AS 0.62) | - |
| Phase E) | Water, demin. | Aqua | 20.00 | 20.00 | 20.00 | 20.00 | 16.00 | 20 |
| | Perfume "Cotton Touch" | Parfum | 0.50 | 0.50 | 0.50 | 0.50 | 0.30 | 0.50 |
| | Sodium Chloride | Sodium Chloride | 0.10 | 1.50 | 1.50 | 1.49 | | 0.50 |
| | Citric Acid (50% solution) | Citric Acid | 0.52 | 0.84 (=AS 0.42) | 0.84 (=AS 0.42) | 0.54 (=AS 0.27) | | 0.75 |
| | Jordapon^{®} SCI | Sodium Cocoyl Isethionate | | | | | 4.0 (AS 3.82) | |
| | Arlypon^{®} TT | PEG/PPG120/10Trimethylolpropane Trioleate and Laureth-2 | | | | | 1.0 | |
| | Plantasil^{®} 4V | PEG-40 Hydrogenated Castor Oil (and) PEG-7 Glyceryl Cocoate (and) PEG/PPG- 120/10 Trimethylolpropan Trioleate (and) Glycerin | | | | | 2.0 | |
| pH value (23 °C) | | | 4.5-4.9 | 4.5-4.9 | 4.5-4.9 | 4.5-4.9 | 4.5-4.9 | 4.5-4.9 |
| Storage stability (4 weeks at 40 °C) | | | ++ | ++ | ++ | ++ | ++ | No (**) |

| | | | | | | | | |
|---|---|---|---|---|---|---|---|---|
| ++ means: stable; no separation; No (**) = separation after 1 week | | | | | | | | |

Shower creams with concentrates according to Example 1 show significantly higher storage stability than the shower cream without a stabilizer concentrate (comparison example 2).

### Example 3: Creamy Care Body Wash

The body wash composition was prepared by mixing ingredients of phase A under stirring and allowed to swell for 10 minutes. The ingredients of phase B were mixed in the given order and stirred until completely homogenous. Phase A was added to Phase B and the ingredients of Phase C were added step by step. The pH was adjusted by adding lactic acid in D).

| **Example 3** | | | |
|---|---|---|---|
| | **Product name** | **INCI/active Ingredient** | **% by weight (%AS)** |
| **Phase A** | Dehyquart^{®} Guar N | Guar Hydroxypropyltrimonium Choride | 0.30 (AS 0.27) |
| | Lactic Acid | Lactic Acid | 0.38 |
| | Water | Aqua | 15.00 |
| **Phase B** | Texapon^{®} N 70 | Sodium Laureth Sulfate | 14.30 (AS 10.01) |
| | Dehyton^{®} PK 45 | Cocoamidopropyl Betaine | 7.50 (AS 2.78) |
| | Lamesoft^{®} PO 65 | Coco-Glucoside, Glyceryloleate | 4.00 |
| | Cetiol^{®} LDO | Dicaprylyl Ether, Lauryl Alcohol | 0.40 |
| | Covi-ox^{®} T 90 C | Tocopherol | 0.02 |
| | Glycerin | Glycerin | 2.50 |
| | D-Panthenol 75 W | Panthenol | 0.50 |
| | Perfume | Parfum | 1.00 |
| | Water | Aqua | 48.90 |
| **Phase C** | Wax Dispersion WD1 | See above | 3.00 |
| | Concentrates Table 1b | | 1.50 (AS 0.62) |
| | Sodium Benzoate | Sodium Benzoate | 0.70 |
| **Phase D** | Lactic Acid | Lactic Acid | qs |
| pH value (23 °C) | | | 4.4-4.8 |
| Viscosity | | | ∼ 19000 mPa s |

The body was cream was white and stable for more than 4 weeks.

### Example 4: Opacified Bodywash

Ingredients of phase A were mixed in given order until completely homogeneous. pH was adjusted by adding phase B and viscosity by adding phase C.

| **Example 4** | **Product name** | **INCI / active Ingredient** | **% by weight (%AS)** |
|---|---|---|---|
| **Phase A** | Water, demin. | Aqua | 72.16 |
| | Texapon^{®} N 70 | Sodium Laureth Sulfate | 14.30 |
| | Dehyton^{®} PK 45 | Cocamidopropyl Betaine | 5.40 |
| | Concentrates Table 1b | see above | 1.50 |
| | Wax dispersion WD1 | see above | 2.00 |
| | Lamesoft^{®} PO 65 | Coco-Glucoside, Glyceryl Oleate | 2.00 |
| | Perfume Cotton Touch (Symrise) | Parfum | 0.50 |
| | Sodium Benzoate | Sodium Benzoate | 0.50 |
| **Phase B** | Citric Acid (50% solution) | Citric Acid | 0.73 |
| **Phase C** | Sodium Chloride | Sodium Chloride | 0.91 |
| ph Value (23°) | | | 4.6 |
| Viscosity (Brookfield; RVT; spindle 4; 10 rpm; 23°C) | | | 12800 mPa s |

### Example 5: Opacified Shampoo

The shampoo was prepared in the same manner was described in example 4.

| **Example 5** | **Product name** | **INCI / active Ingredient** | **% by weight (%AS)** |
|---|---|---|---|
| **Phase A** | Water, demin. | Aqua | 74.69 |
| | Dehyquart^{®} Guar N | Guar Hydroxypropyltrimonium Chloride | 0.20 |
| | Citric Acid (50% solution) | Citric Acid | 0.05 |
| **Phase B** | Texapon^{®} N 70 | Sodium Laureth Sulfate | 14.30 |
| | Dehyton^{®} PK 45 | Cocamidopropyl Betaine | 4.16 |
| | Concentrates Table 1b | see above | 1.50 |
| | Wax Dispersion 1 | see above | 2.20 |
| | Perfume Cotton Touch (Symrise) | Parfum | 0.50 |
| | Sodium Benzoate | Sodium Benzoate | 0.50 |
| **Phase C** | Citric Acid (50% solution) | Citric Acid | 0.60 |
| **Phase D** | Sodium Chloride | Sodium Chloride | 1.30 |
| ph Value (23°) | | | 4.7 |
| Viscosity (Brookfield; RVT; spindle 4; 10 rpm; 23°C) | | | 6800 mPa s |

### Example 6: Opacified Shampoo

Ingredients of phase A were mixed under stirring and allowed to swell for approx. 10 minutes. Ingredients of phase B were added in given order and stirred until completely homogeneous. By adding phase C pH was adjusted and viscosity by adding phase D.

| **Example 6** | **Product name** | **INCI / active Ingredient** | **% by weight (%AS)** |
|---|---|---|---|
| **Phase A** | Water, demin. | Aqua | 73.99 |
| | Dehyquart^{®} Guar N | Guar Hydroxypropyltrimonium Chloride | 0.20 |
| | Citric Acid (50% solution) | Citric Acid | 0.05 |
| **Phase B** | Texapon^{®} N 70 | Sodium Laureth Sulfate | 14.30 |
| | Dehyton^{®} PK 45 | Cocamidopropyl Betaine | 4.16 |
| | Concentrates Table 1b | see above | 1.50 |
| | Lamesoft^{®} TM Benz | Glycol Distearate, Coco-Glucoside, Glyceryl Oleate, Glyceryl Stearate | 3.00 |
| | Perfume Cotton Touch (Symrise) | Parfum | 0.50 |
| | Sodium Benzoate | Sodium Benzoate | 0.50 |
| **Phase C** | Citric Acid (50% solution) | Citric Acid | 0.60 |
| **Phase D** | Sodium Chloride | Sodium Chloride | 1.20 |
| pH Value (23°) | | | 4.7 |
| Viscosity (Brookfield; RVT; spindle 4; 10 rpm; 23°C) | | | 8300 mPa s |

### Example 7: Conditioning Shampoo

Ingredients of phase A were mixed under stirring and allowed to swell for approx. 10 minutes. Ingredients of phase B were mixed in given order and stirred after each addition until completely homogeneous. Phase A was added to phase B and afterwards phase C was added step by step. PH was adjusted by adding phase D and viscosity by adding phase E.

| **Example 7** | **Product name** | **INCI / active Ingredient** | **% by weight (%AS)** |
|---|---|---|---|
| **Phase A** | Water, demin. | Aqua | 15.00 |
| | Dehyquart^{®} Guar N | Guar Hydroxypropyltrimonium Chloride | 0.30 |
| | Citric Acid (50% solution) | Citric Acid | 0.10 |
| **Phase B** | Texapon^{®} N 70 | Sodium Laureth Sulfate | 12.86 |
| | Dehyton^{®} PK 45 | Cocamidopropyl Betaine | 8.10 |
| | Perfume Cotton Touch (Symrise) | Parfum | 0.50 |
| | Water, demin. | Aqua | 57.14 |
| **Phase C** | Concentrates Table 1b | see above | 1.50 |
| | Wax Dispersion 1 | see above | 2.00 |
| | Wax Dispersion 3 | see above | 2.00 |
| | Sodium Benzoate | Sodium Benzoate | 0.50 |
| **Phase D** | Citric Acid (50% solution) | Citric Acid | q.s. |
| **Phase E** | Sodium Chloride | Sodium Chloride | q.s. |
| pH Value (23°) | | | 4.6-4.8 |
| Viscosity (Brookfield; RVT; spindle 4; 10 rpm; 23°C) | | | ~ 6000 mPa s |

## Claims

1. Stabilizer concentrates for waxes selected from the group consisting of mono- and/or diesters of mono- and/or diethylene glycol, whereby the waxes are in form of a dispersion comprised in personal care compositions, and wherein the concentrates are containing
(a) hydrogenated castor oil,
(b) nonionic emulsifiers selected from the group consisting of fatty alcohol polyglycol ether, fatty acid polyglycol ester; ethoxylated triglycerides, ethoxylated fatty acid glycerol ester; mixed ethers or mixed formals; polysorbates and sugar-based carbohydrates
(c) optional amphoteric and/or zwitterionic surfactants
(d) optional other auxiliaries or additives, including salts, and
(e) water,
with the provisos that the concentrates are free from anionic surfactants and contain hydrogenated castor oil a) in a quantity above 7,0 to 20 wt% and wherein the sum of quantities of (a) to (d) is in the range from 30% to 80% by weight and add up to 100% by weight by water.

2. Concentrates as claimed in claim 1, **characterized in that** they contain (a) hydrogenated castor oil in the quantity range from 7,5 to 15 %, preferred 8,5 to 12%, by weight - based on concentrates.

3. Concentrates as claimed in claim 1 and/or 2, **characterized in that** they contain nonionic emulsifiers b) selected from the group consisting of sugar-based carbohydrates, preferred alk(en)yl polyglycosides, glucuronic acid derivatives and fatty acid N-alkylglucamides and especially alk(en)yl polyglycosides.

4. Concentrates as claimed in at least one claim 1 to 3, **characterized in that** they contain nonionic emulsifiers b) in the quantity range from 10 to 60 % by weight - based on concentrates.

5. Concentrates as claimed in at least one of claims 1 to 4, **characterized in that** the quantity ratio of nonionic emulsifiers b) to hydrogenated castor oil (a) is in the range from 2:1 to 4:1.

6. Concentrates as claimed in at least one of claims 1 to 5, **characterized in that** they contain other auxiliaries or additives, including salts, (d) in the quantity range from 0.1 to 15% by weight - based on concentrates.

7. Concentrates as claimed in at least one of claims 1 to 6, **characterized in that** they contain amphoteric and/or zwitterionic surfactants (c) selected from the group consisting of betaines and/or amphoacetats.

8. Concentrates as claimed in at least one of the claims 1 to 7, **characterized in that** they contain amphoteric and/or zwitterionic surfactants (c) in the quantity range from 0 to 30% by weight, - based on concentrates.

9. Concentrates as claimed in at least one of the claims 1 to 7, **characterized in that** they consist of
(a) above 7,0 to 20 % by weight of hydrogenated castor oil,
(b) 10.0 to 60.0% by weight of nonionic emulsifiers,
(d) 0.1 to 15.0% by weight of other auxiliaries or additives, including salts, and
(e) add up to 100% by weight by water.

10. Concentrates as claimed in at least one of the claims 1 to 9, **characterized in that** they consist of
(a) 7.5 to 15% by weight of hydrogenated castor oil,
(b) 20.0 to 50.0% by weight of nonionic emulsifiers,
(d) 0.1 to 15.0% by weight of other auxiliaries or additives, including salts,
(e) and add up to 100% by weight by water.

11. Concentrates as claimed in at least one of the claims 1 to 10, **characterized in that** they have a viscosity (Brookfield, RVT; spindle 4; 10 rpm; 20°C) in the range of 2.500 to 8.000 mPas, especially 3.000 to 7.000 mPas.

12. Concentrates as claimed in at least one of the claims 1 to 11, **characterized in that** they stabilise waxes comprising 90 to 100 wt% diethylene glycol di fatty acid ester and 0 to 10 wt % diethylene glycol mono fatty acid ester- wt % based on the wax, and particularly preferred ester of a fatty acid mixture, which contains 90 to 98 wt% stearic acid and 2 to 10 wt% other saturated fatty acids with 16 to 22 carbon atoms- wt% based on the fatty acid mixture.

13. Concentrates as claimed in at least one of the claims 1 to 12, **characterized in that** they stabilise waxes having an average particle size d50- measured via laser diffraction by Mastersizer 2000^{®} - from 0.8 to 3.5 µm, especially 1.0 to 3.0 µm.

14. A process for the producing concentrates claimed in at least one of the claims 1 to 11 , **characterized in that** the concentrates are prepared by mixing components (a), (b) and optionally (c) and/or optionally (d) together, adding up with the necessary quantity of water, heating the mixture to about 85 to 95 °C before cooling down with agitation.

15. Use of concentrates as claimed in at least one of the claims 1 to 11 and/or claim 14 as a stabilizer for waxes selected from the group consisting of mono- and/or diesters of mono- and/or diethylene glycol, whereby the waxes are in form of a dispersion comprised in personal care compositions.

16. Use of concentrates as claimed in claim 15, **characterized in that** they are produced according to claim 14.

17. Use of concentrates as claimed in at least one of the claims 15, **characterized in that** the concentrates are stabilizers for waxes, which are used in form of a wax dispersion as a pearlizing and/or opacifier agent in personal care compositions, preferable in personal care compositions comprising cationic polymers.

18. Personal care compositions comprising
A) 0.1wt% to 5 wt%, especially 0.25 wt% to 3 wt% of the concentrates as claimed in at least one of the claims 1 to 13 and
B) wax dispersions comprising waxes selected from the group consisting of mono- and/or diesters of mono- and/or diethylene glycol and
C) one or more detersive surfactants selected from the groups selected from anionic surfactants, nonionic surfactants, amphoteric or zwitterionic surfactants and
D) optional cationic polymers and
E) optional other component(s) different from C) and/or D).

19. Process for producing personal care compositions as claimed in claim 18, **characterized in that** one or more surfactants C) and the wax dispersion B) and optional cationic polymers D) are mixed at room temperature, and the concentrates A) as claimed in at least one of the claims 1 to 13 are added and stirred together, wherein the optionally other components E) can be added before or after the addition of concentrates A).

## Patentansprüche

1. Stabilisatorkonzentrate für Wachse, ausgewählt aus der Gruppe bestehend aus Mono- und/oder Diestern von Mono- und/oder Diethylenglykol, wobei die Wachse in Form einer in Körperpflegezusammensetzungen enthaltenen Dispersion vorliegen und wobei die Konzentrate
(a) hydriertes Ricinusöl,
(b) nichtionische Emulgatoren aus der Gruppe bestehend aus Fettalkoholpolyglykolether, Fettsäurepolyglykolester, ethoxylierten Triglyceriden, ethoxyliertem Fettsäureglycerinester, Mischethern oder Mischformalen, Polysorbaten und zuckerbasierten Kohlenhydraten,
(c) fakultative amphotere und/oder zwitterionische Tenside,
(d) fakultative weitere Hilfsstoffe oder Additive einschließlich Salzen und
(e) Wasser
enthalten,
mit den Maßgaben, dass die Konzentrate frei von anionischen Tensiden sind und hydriertes Ricinusöl a) in einer Menge von mehr als 7,0 bis 20 Gew.-% enthalten und wobei die Summe der Mengen von (a) bis (d) im Bereich von 30 bis 80 Gew.-% liegt und sich mit Wasser zu 100 Gew.-% summieren.

2. Konzentrate nach Anspruch 1, **dadurch gekennzeichnet, dass** sie (a) hydriertes Ricinusöl im Mengenbereich von 7,5 bis 15 Gew.-%, bevorzugt 8,5 bis 12 Gew.-%, bezogen auf die Konzentrate, enthalten.

3. Konzentrate nach Anspruch 1 und/oder 2, **dadurch gekennzeichnet, dass** sie nichtionische Emulgatoren b) enthalten, die aus der Gruppe bestehend aus zuckerbasierten Kohlenhydraten, bevorzugt Alk(en)ylpolyglykosiden, Glucuronsäurederivaten und Fettsäure-N-alkylglucamiden und insbesondere Alk(en)ylpolyglykosiden ausgewählt sind.

4. Konzentrate nach mindestens einem der Ansprüche 1 bis 3, **dadurch gekennzeichnet, dass** sie nichtionische Emulgatoren b) im Mengenbereich von 10 bis 60 Gew.-%, bezogen auf die Konzentrate, enthalten.

5. Konzentrate nach mindestens einem der Ansprüche 1 bis 4, **dadurch gekennzeichnet, dass** das Mengenverhältnis von nichtionischen Emulgatoren b) zu hydriertem Ricinusöl (a) im Bereich von 2:1 bis 4:1 liegt.

6. Konzentrate nach mindestens einem der Ansprüche 1 bis 5, **dadurch gekennzeichnet, dass** sie weitere Hilfsstoffe oder Additive einschließlich Salzen (d) im Mengenbereich von 0,1 bis 15 Gew.-%, bezogen auf die Konzentrate, enthalten.

7. Konzentrate nach mindestens einem der Ansprüche 1 bis 6, **dadurch gekennzeichnet, dass** sie amphotere und/oder zwitterionische Tenside (c) enthalten, die aus der Gruppe bestehend aus Betainen und/oder Amphoacetaten ausgewählt sind.

8. Konzentrate nach mindestens einem der Ansprüche 1 bis 7, **dadurch gekennzeichnet, dass** sie amphotere und/oder zwitterionische Tenside (c) im Mengenbereich von 0 bis 30 Gew.-%, bezogen auf die Konzentrate, enthalten.

9. Konzentrate nach mindestens einem der Ansprüche 1 bis 7, **dadurch gekennzeichnet, dass** sie aus
(a) mehr als 7,0 bis 20 Gew.-% hydriertem Ricinusöl,
(b) 10,0 bis 60,0 Gew.-% nichtionischen Emulgatoren,
(d) 0,1 bis 15,0 Gew.-% weiteren Hilfsstoffen oder Additiven einschließlich Salzen bestehen und
(e) sich mit Wasser zu 100 Gew.-% summieren.

10. Konzentrate nach mindestens einem der Ansprüche 1 bis 9, **dadurch gekennzeichnet, dass** sie aus
(a) 7,5 bis 15 Gew.-% hydriertem Ricinusöl,
(b) 20,0 bis 50,0 Gew.-% nichtionischen Emulgatoren,
(d) 0,1 bis 15,0 Gew.-% weiteren Hilfsstoffen oder Additiven einschließlich Salzen bestehen und
(e) sich mit Wasser zu 100 Gew.-% summieren.

11. Konzentrate nach mindestens einem der Ansprüche 1 bis 10, **dadurch gekennzeichnet, dass** sie eine Viskosität (Brookfield, RVT; Spindel 4; 10 U/min; 20 °C) im Bereich von 2500 bis 8000 mPas, insbesondere 3000 bis 7000 mPas, aufweisen.

12. Konzentrate nach mindestens einem der Ansprüche 1 bis 11, **dadurch gekennzeichnet, dass** sie Wachse stabilisieren, die 90 bis 100 Gew.-% Diethylenglykoldifettsäureester und 0 bis 10 Gew.-% Diethylenglykolmonofettsäureester, wobei sich die Gew.-%-Angaben auf das Wachs beziehen, und besonders bevorzugt Ester eines Fettsäuregemischs, das 90 bis 98 Gew.-% Stearinsäure und 2 bis 10 Gew.-% andere Fettsäuren mit 16 bis 22 Kohlenstoffatomen, wobei sich die Gew.-%-Angaben auf das Fettsäuregemisch beziehen, enthält, umfassen.

13. Konzentrate nach mindestens einem der Ansprüche 1 bis 12, **dadurch gekennzeichnet, dass** sie Wachse mit einer mittels Laserbeugung mit dem Mastersizer 2000^{®} gemessenen durchschnittlichen Teilchengröße d50 von 0,8 bis 3,5 µm, insbesondere 1,0 bis 3,0 µm, stabilisieren.

14. Verfahren zur Herstellung der Konzentrate nach mindestens einem der Ansprüche 1 bis 11, **dadurch gekennzeichnet, dass** die Konzentrate durch Mischen der Komponenten (a), (b) und gegebenenfalls (c) und/oder gegebenenfalls (d) miteinander, Zugeben der erforderlichen Menge Wasser, Erhitzen der Mischung auf etwa 85 bis 95 °C und anschließendes Abkühlen unter Rühren hergestellt werden.

15. Verwendung von Konzentraten nach mindestens einem der Ansprüche 1 bis 11 und/oder Anspruch 14 als Stabilisator für Wachse, die aus der Gruppe bestehend aus Mono- und/oder Diestern von Mono- und/oder Diethylenglykol ausgewählt sind, wobei die Wachse in Form einer in Körperpflegezusammensetzungen enthaltenen Dispersion vorliegen.

16. Verwendung von Konzentraten nach Anspruch 15, **dadurch gekennzeichnet, dass** sie gemäß Anspruch 14 hergestellt werden.

17. Verwendung von Konzentraten nach mindestens einem der Ansprüche 15, **dadurch gekennzeichnet, dass** es sich bei den Konzentraten um Stabilisatoren für Wachse handelt, die in Form einer Wachsdispersion als Perlglanz- und/oder Trübungsmittel in Körperpflegezusammensetzungen, vorzugsweise in Körperpflegezusammensetzungen, die kationische Polymere umfassen, verwendet werden.

18. Körperpflegezusammensetzungen, umfassend
A) 0,1 Gew.-% bis 5 Gew.-%, insbesondere 0,25 Gew.-% bis 3 Gew.-%, der Konzentrate nach mindestens einem der Ansprüche 1 bis 13 und
B) Wachsdispersionen, die Wachse umfassen, die aus der Gruppe bestehend aus Mono- und/oder Diestern von Mono- und/oder Diethylenglykol ausgewählt sind, und
C) ein oder mehrere waschaktive Tenside aus den Gruppen, die aus anionischen Tensiden, nichtionischen Tensiden, amphoteren oder zwitterionischen Tensiden ausgewählt sind, und
D) fakultative kationische Polymere und
E) eine oder mehrere fakultative weitere Komponenten, die von C) und/oder D) verschieden sind.

19. Verfahren zur Herstellung von Körperpflegezusammensetzungen nach Anspruch 18, **dadurch gekennzeichnet, dass** man das eine oder die mehreren Tenside C) und die Wachsdispersion B) und fakultative kationische Polymere D) bei Raumtemperatur mischt und Konzentrate A) nach mindestens einem der Ansprüche 1 bis 13 zugibt und zusammenrührt, wobei die fakultativen weiteren Komponenten E) vor oder nach der Zugabe der Konzentrate A) zugegeben werden können.

## Revendications

1. Concentrés de stabilisant pour des cires choisies dans le groupe constitué par des monoesters et/ou diesters de monoéthylèneglycol et/ou diéthylèneglycol, les cires étant sous forme d'une dispersion comprise dans des compositions de soins personnels, et les concentrés contenant
(a) de l'huile de ricin hydrogénée,
(b) des émulsifiants non ioniques choisis dans le groupe constitué par un éther de polyglycol et d'alcool gras, un ester de polyglycol et d'acide gras ; des triglycérides éthoxylés, un ester de glycérol et d'acide gras éthoxylé ; des éthers mixtes ou des formals mixtes ; des polysorbates et des glucides à base de sucre
(c) des tensioactifs amphotères et/ou zwitterioniques facultatifs
(d) d'autres auxiliaires ou additifs facultatifs, notamment des sels, et
(e) de l'eau,
à condition que les concentrés soient exempts de tensioactifs anioniques et contiennent de l'huile de ricin hydrogénée a) en une quantité de plus de 7,0 à 20 % en poids et dans lesquels la somme des quantités de (a) à (d) est dans la plage allant de 30 % à 80 % en poids et est complétée à 100 % en poids avec de l'eau.

2. Concentrés tels que revendiqués dans la revendication 1, **caractérisés en ce qu'**ils contiennent (a) de l'huile de ricin hydrogénée dans la plage de quantité allant de 7,5 à 15 %, de préférence de 8,5 à 12 %, en poids, sur la base des concentrés.

3. Concentrés tels que revendiqués dans la revendication 1 et/ou 2, **caractérisés en ce qu'**ils contiennent des émulsifiants non ioniques b) choisis dans le groupe constitué par des glucides à base de sucre, de préférence des (alkyl ou alcényl)polyglycosides, des dérivés d'acide glucuronique et des N-alkylglucamides d'acides gras et en particulier des (alkyl ou alcényl)polyglycosides.

4. Concentrés tels que revendiqués dans au moins l'une des revendications 1 à 3, **caractérisés en ce qu'**ils contiennent des émulsifiants non ioniques b) dans la plage de quantité allant de 10 à 60 % en poids, sur la base des concentrés.

5. Concentrés tels que revendiqués dans au moins l'une des revendications 1 à 4, **caractérisés en ce que** le rapport des quantités des émulsifiants non ioniques b) à l'huile de ricin hydrogénée (a) est dans la plage allant de 2:1 à 4:1.

6. Concentrés tels que revendiqués dans au moins l'une des revendications 1 à 5, **caractérisés en ce qu'**ils contiennent d'autres auxiliaires ou additifs, notamment des sels, (d) dans la plage de quantité allant de 0,1 à 15 % en poids, sur la base des concentrés.

7. Concentrés tels que revendiqués dans au moins l'une des revendications 1 à 6, **caractérisés en ce qu'**ils contiennent des tensioactifs amphotères et/ou zwitterioniques (c) choisis dans le groupe constitué par des bétaïnes et/ou des amphoacétates.

8. Concentrés tels que revendiqués dans au moins l'une des revendications 1 à 7, **caractérisés en ce qu'**ils contiennent des tensioactifs amphotères et/ou zwitterioniques (c) dans la plage de quantité allant de 0 à 30 % en poids, sur la base des concentrés.

9. Concentrés tels que revendiqués dans au moins l'une des revendications 1 à 7, **caractérisés en ce qu'**ils sont constitués de
(a) plus de 7,0 à 20 % en poids d'huile de ricin hydrogénée,
(b) 10,0 % 60,0 % en poids d'émulsifiants non ioniques,
(d) 0,1 à 15,0 % en poids d'autres auxiliaires ou additifs, notamment des sels, et
(e) le complément à 100 % en poids avec de l'eau.

10. Concentrés tels que revendiqués dans au moins l'une des revendications 1 à 9, **caractérisés en ce qu'**ils sont constitués de
(a) 7,5 à 15 % en poids d'huile de ricin hydrogénée,
(b) 20,0 % 50,0 % en poids d'émulsifiants non ioniques,
(d) 0,1 à 15,0 % en poids d'autres auxiliaires ou additifs, notamment des sels,
(e) et le complément à 100 % en poids avec de l'eau.

11. Concentrés tels que revendiqués dans au moins l'une des revendications 1 à 10, **caractérisés en ce qu'**ils ont une viscosité (Brookfield, RVT ; mobile 4 ; 10 tr/min ; 20 °C) dans la plage de 2 500 à 8 000 mPa.s, en particulier de 3 000 à 7 000 mPa.s.

12. Concentrés tels que revendiqués dans au moins l'une des revendications 1 à 11, **caractérisés en ce qu'**ils stabilisent des cires comprenant 90 à 100 % en poids de diester d'acide gras et de diéthylèneglycol et 0 à 10 % en poids de monoester d'acide gras et de diéthylèneglycol, les % en poids étant sur la base de la cire, et de préférence surtout un ester d'un mélange d'acides gras, qui contient 90 à 98 % en poids d'acide stéarique et 2 à 10 % en poids d'autres acides gras saturés ayant 16 à 22 atomes de carbone, les % en poids étant sur la base du mélange d'acides gras.

13. Concentrés tels que revendiqués dans au moins l'une des revendications 1 à 12, **caractérisés en ce qu'**ils stabilisent des cires ayant une taille moyenne des particules d50, mesurée par le biais de la diffraction laser par Mastersizer 2000^{®}, allant de 0,8 à 3,5 µm, en particulier de 1,0 à 3,0 pm.

14. Procédé pour la production de concentrés revendiqués dans au moins l'une des revendications 1 à 11, **caractérisé en ce que** les concentrés sont préparés par mélange des composants (a), (b) et facultativement (c) et/ou facultativement (d) les uns avec les autres, ajout de la quantité d'eau nécessaire pour compléter, chauffage du mélange à environ 85 à 95 °C avant refroidissement sous agitation.

15. Utilisation de concentrés tels que revendiqués dans au moins l'une des revendications 1 à 11 et/ou la revendication 14 en tant que stabilisant pour des cires choisies dans le groupe constitué par des monoesters et/ou diesters de monoéthylèneglycol et/ou diéthylèneglycol, les cires étant sous forme d'une dispersion comprise dans des compositions de soins personnels.

16. Utilisation de concentrés telle que revendiquée dans la revendication 15, **caractérisée en ce qu'**ils sont produits selon la revendication 14.

17. Utilisation de concentrés telle que revendiquée dans au moins l'une des revendications 15, **caractérisée en ce que** les concentrés sont des stabilisants pour des cires, qui sont utilisées sous forme d'une dispersion de cires en tant qu'agent nacrant et/ou opacifiant dans des compositions de soins personnels, de préférence dans des compositions de soins personnels comprenant des polymères cationiques.

18. Compositions de soins personnels comprenant
A) 0,1 % en poids à 5 % en poids, en particulier 0,25 % en poids à 3 % en poids des concentrés tels que revendiqués dans au moins l'une des revendications 1 à 13 et
B) des dispersions de cires comprenant des cires choisies dans le groupe constitué par des monoesters et/ou diesters de monoéthylèneglycol et/ou diéthylèneglycol et
C) un ou plusieurs tensioactifs détersifs choisis dans les groupes choisis parmi des tensioactifs anioniques, des tensioactifs non ioniques, des tensioactifs amphotères ou zwitterioniques et
D) des polymères cationiques facultatifs et
E) un ou plusieurs autres composants facultatifs différents de C) et/ou D).

19. Procédé pour la production de compositions de soins personnels telles que revendiquées dans la revendication 18, **caractérisé en ce qu'**un ou plusieurs tensioactifs C) et la dispersion de cires B) et des polymères cationiques facultatifs D) sont mélangés à température ambiante et les concentrés A) tels que revendiqués dans au moins l'une des revendications 1 à 13 sont ajoutés et agités ensemble, dans lequel les autres composants facultatifs E) peuvent être ajoutés avant ou après l'ajout de concentrés A).
